(19) Europäisches Patentamt
European Patent Office
Office européen des brevets

(11) **EP 3 235 432 A1**

(12) # EUROPEAN PATENT APPLICATION

(43) Date of publication:
**25.10.2017 Bulletin 2017/43**

(51) Int Cl.:
***A61B 6/00*** (2006.01)

(21) Application number: **16165956.0**

(22) Date of filing: **19.04.2016**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Agfa HealthCare N.V.**
**2640 Mortsel (BE)**

(72) Inventors:
• **NEBOSIS, Rainer**
**81541 München (DE)**
• **ZEMÁNEK, Vladimír**
**85579 Neubiberg (DE)**

(54) **MOBILE RADIATION IMAGE CAPTURING SYSTEM AND METHOD**

(57)   The invention relates to a mobile radiation image capturing system comprising : an X-ray radiation generation unit mounted on a mobile carriage; a drive unit configured to move the carriage over the floor by linear motion in two dimensions and/or by rotation; at least one radiation image detection unit; at least one sensor unit (10, 11) configured to determine an orientation and/or a position of the radiation generation unit and the detection unit relative to each other, a processing unit (30) configured to determine whether the radiation generation unit and the detection unit have a predetermined orientation and/or position relative to each other and, in the negative, how the carriage and/or the radiation generation unit has to be moved, and to control an output unit (31) to output the determined positioning information and/or to control the drive unit (33) to move the carriage.

Fig. 3

**Description**

The present invention relates to a mobile radiation image capturing system and a method for operating a mobile radiation image capturing system according to the independent claims.

In medical imaging, in particular in X-ray imaging, it is usually necessary to position a patient's body or body part with respect to imaging means, in particular with respect to an X-ray radiation generation unit and an X-ray radiation detection unit. In the case of very sick or immobile patients, mobile imaging systems are used by which the imaging means can be positioned relative to the patient. In order to ensure a defined imaging geometry, the radiation generation unit and the detection unit have to be positioned relative to each other.

It is an object of the invention to provide a mobile radiation image capturing system and an according method for operating a mobile radiation image capturing system which allow for a reliable positioning of the radiation generation unit and the detection unit relative to each other.

This object is achieved by the system and method according to the independent claims.

A mobile radiation image capturing system according to an aspect of the invention comprises a radiation generation unit configured to generate X-ray radiation, a mobile carriage, on which the radiation generation unit is mounted, and a drive unit configured to move the carriage over the floor by linear motion in two dimensions and/or by rotation. Further, the system comprises at least one detection unit configured to capture a radiation image of an object based on X-ray radiation generated by the radiation generation unit and transmitted and/or reflected by the object, and at least one sensor unit configured to determine an orientation and/or a position of the radiation generation unit and the detection unit relative to each other. The system further comprises at least one output unit configured to output information to a user, and a processing unit configured to determine, based on the orientation and/or position of the radiation generation unit and the detection unit relative to each other, whether the radiation generation unit and the detection unit have a predetermined orientation and/or position relative to each other and, in the negative, to determine a positioning information on how the carriage and/or the radiation generation unit has to be moved in order to bring the radiation generation unit and the detection unit into a predetermined orientation and/or position relative to each other, and to control the output unit to output the determined positioning information and/or to control the drive unit to move the carriage based on the determined positioning information.

A method according to another aspect of the invention allows for operating a mobile radiation image capturing system, wherein the system comprises a radiation generation unit configured to generate X-ray radiation, a mobile carriage on which the radiation generation unit is mounted, a drive unit configured to move the carriage over the floor, and at least one detection unit configured to capture a radiation image of an object based on X-ray radiation generated by the radiation generation unit and transmitted and/or reflected by the object, and the method comprises the following steps: determining an orientation and/or a position of the radiation generation unit and the detection unit relative to each other, determining, based on the orientation and/or position of the radiation generation unit and the detection unit relative to each other, whether the radiation generation unit and the detection unit have a predetermined orientation and/or position relative to each other and, in the negative, determining a positioning information on how the carriage and/or the radiation generation unit has to be moved in order to bring the radiation generation unit and the detection unit into a predetermined orientation and/or position relative to each other, and outputting the positioning information via an output unit to a user and/or controlling the drive unit to move, based on the positioning information, the carriage over the floor by linear motion in two dimensions and/or by rotation.

The invention is based on the approach to determine whether the radiation generation unit and the detection unit are in a predetermined orientation and/or position relative to each other. A predetermined orientation and/or position may be, e.g., an orientation and/or position of the radiation generation unit and the detection unit, wherein a center beam of the radiation generated by the radiation generation unit impinges almost perpendicularly, or under a predefined angle close to 90°, onto a central area of the detection unit and/or wherein a beam cone of the generated radiation lies within the area of the detection unit. In case that the radiation generation unit and the detection unit are not in the predetermined orientation and/or position relative to each other, a positioning information is determined by considering the current orientation and/or position and the predefined orientation and/or position of the radiation generation unit and the detection unit. The current orientation and/or position of the radiation generation unit and the detection unit is determined based on signals generated by at least one sensor unit which is provided at the radiation generation unit and/or the detection unit. The positioning information relates to information on how a mobile carriage, on which the radiation generation unit is mounted, has to be moved or positioned in order to bring the radiation generation unit and the detection unit into the predetermined orientation and/or position relative to each other. To this end, the mobile carriage is configured to be moved across the floor by linear motion and/or rotation, in particular by rotation in place, by means of a drive unit which is configured to drive the carriage accordingly and/or by manual force of a user applied to the carriage and/or the radiation generation unit. In the former case, the drive unit is controlled to move or position the carriage such that the radiation generation unit and the detection unit are brought into the predetermined orientation and/or position relative to each other based on the positioning information and/or based on movement instructions which are derived from the positioning information. Alternatively or additionally, the positioning information is outputted via an output device, e.g. a display or loudspeaker, to the user who can, by considering the positioning information, move the carriage and/or the radiation generation unit and/or the detector relative to each other, e.g. manually or by inputting corresponding movement instructions based on which the drive unit is controlled to move the carriage accordingly.

By this means, the radiation generation unit and the detection unit of a mobile radiation image capturing system can be easily and reliably brought into a predetermined orientation and/or position relative to each other.

Preferably, the carriage comprises wheels which are configured to allow for a linear movement, e.g. a forward and/or sideward movement of the carriage across the floor, and/or a rotation, in particular a rotation on the spot, of the carriage. In particular, at least two wheels are designed as so-called omnidirectional wheels, also referred to as "omni wheels" or "poly wheels", which can be driven or rotated along a forward direction but also laterally. Further preferably, the at least two wheels are omni wheels with two drives, wherein each of the omni wheels consists of a larger first wheel which is configured for driving a forward movement by rotation around a first main axis and a second smaller wheel which is configured for driving a sideward movement or a rotation of the carriage by rotation around a second main axis, and wherein the first larger wheel and the second smaller wheel are arranged one after another, such that the first main axis and the second main axis are perpendicular to one another and parallel to the floor, respectively. Alternatively or additionally, at least one of the at least two wheels may be configured as a mecanum wheel. Alternatively or additionally, at least two wheels of the carriage are designed as caster wheels which can be driven or rotated along a forward direction and are rotatable around a vertical axis. In each case, a particularly mobile, i.e. maneuverable, carriage is provided such that the predetermined orientation and/or position of the radiation generation unit and the detection unit relative to each other can be adjusted easily by moving the carriage accordingly. This is of particular advantage in bedside applications, where space is usually limited.

In a preferred embodiment, the drive unit is and/or the wheels of the carriage, in particular the omni wheels, are configured to allow for a rotation of the carriage around different rotation centers. Further preferably, the different rotation centers are selectable by a user. For example, a first rotation center is located at the front tip of the carriage. Alternatively or additionally, a second rotation center is located on the axis of a column to which the radiation generation unit is mounted to. Further alternatively or additionally, a third rotation center is located in the center of mass of the carriage. Further preferably, the radiation image capturing system comprises means for a user to choose from a list of different rotation centers or to customize new rotation centers, for example via a control element. By this means, the movement of the carriage for bringing the radiation generation unit and the detection unit in a predetermined position and/or orientation relative to each other can be easily adapted to the present spatial arrangement of the carriage, the patient's bed or other hospital furniture, which is particularly advantageous in places where space is limited.

According to another preferred embodiment of the invention, the mobile radiation image capturing system comprises at least one control element configured to receive movement instructions from the user, wherein the processing unit is configured to control the drive unit to move the carriage based on the movement instructions received from the user. In cases where, for instance, a direct route to a new position is obstructed, the user may maneuver the mobile carriage and/or the radiation generation unit around the obstacle by inputting according movement instructions into the control element. By this means, the movement and/or positioning of the mobile carriage becomes particularly reliable and flexible so that a reliable and exact positioning of the radiation generation unit and the detection unit is achieved.

In another preferred embodiment, the at least one control element is located at the radiation generation unit, in particular at a housing enclosing the radiation generation unit. In this way, the user is enabled to position the radiation generation unit particularly precisely with respect to the detection unit via a movement of the carriage.

Preferably, the at least one output unit is located close to the at least one control element and/or at the radiation generation unit, in particular at the housing enclosing the radiation generation unit. By this means, the user is enabled to comfortably and reliably perceive or obtain the determined positioning information and to control the movement of the carriage via inputting according movement instructions via the control unit, so that the radiation generation unit can be particularly precisely positioned with respect to the detection unit via a movement of the carriage.

Preferably, the output unit is configured to output a signal to the user when the radiation generation unit and the detection unit have reached the predetermined orientation and/or position.

Further preferably, the processing unit is configured to update the positioning information which is output at the output unit according to the movement and/or positioning of the mobile carriage. By this means, the user receives feedback continuously on how his control of the at least one control element has changed the orientation and/or position of radiation generation unit and detection unit relative to each other. By this means, the radiation generation unit and the detection unit can be brought into the predetermined position and/or orientation particularly reliably.

Preferably, the output unit is designed as a touch-sensitive display or screen so that information can be both displayed and inputted. It may be further preferred that the at least one control element is integrated in the output unit so that movement instructions for moving and/or positioning the mobile carriage can be inputted via the touch-sensitive display or screen of the output unit.

In another preferred embodiment, the system further comprises a first sensor unit provided at the radiation generation unit and a second sensor unit provided at the detection unit, wherein each of the first and second sensor unit comprises at least one accelerometer sensor configured to capture first information regarding an acceleration of the accelerometer sensor with respect to three spatial directions, and/or at least one gyroscope sensor configured to capture second information regarding an orientation of the gyroscope sensor with respect to the three spatial directions, and/or at least one magnetic field sensor configured to capture third information regarding a magnetic field surrounding the sensor along the three spatial directions. Preferably, the processing unit is configured to determine the orientation and/or position of the radiation generation unit and the detection unit relative to each other by considering at least one of the first, second and third information captured by the accelerometer, gyroscope or magnetic field sensor, respectively, of the first sensor unit provided at the radiation generation unit, and at least one of the first, second and third information captured by the accelerometer, gyroscope and magnetic field sensor, respectively, of the second sensor unit provided at the detection unit. Preferably, the processing unit is configured to determine whether the radiation generation unit and the detection unit are in a predetermined orientation and/or position relative to each other based on the first and/or second and/or third information captured by the accelerometer, gyroscope or magnetic field sensor, respectively of the first sensor unit and/or the second sensor unit.

By this means, a particularly reliable and precise determination of the orientation and/or the position of the radiation generation unit and detection unit relative to each other is achieved.

Preferably, each of the sensor units comprises at least one acceleration sensor and at least one gyroscope sensor and at least one magnetic field sensor. Further preferably, each of the acceleration sensor, the gyroscope sensor and the magnetic field sensor is a 3D or 3-axis sensors, i.e. each of the sensors is configured to measure an acceleration, an inclination and a magnetic field, respectively, with respect to three spatial directions. Therefore, both the first and the second sensor unit can be regarded as a "9-axis" sensor unit.

The acceleration sensor is configured to determine the tilt of the radiation generation unit or the detection unit, respectively, with respect to the gravity vector if the radiation generation unit or the detection unit is not accelerated. In this case, the acceleration sensor provides an absolute inclination of the radiation generation unit or the detection unit, respectively. However, the inclination information provided by the acceleration sensor can be influenced by an acceleration of the acceleration sensor due to movement.

The gyroscope sensor is configured to determine a tilt with respect to a defined axis of the gyroscope sensor such that it is configured to determine a change in the inclination of the radiation generation unit or the detection unit, respectively. Accordingly, the gyroscope sensor provides a relative inclination of radiation generation unit or detection unit. Nevertheless, based on one or more values of the relative inclination an absolute inclination of the gyroscope sensor can be derived provided that the gyroscope sensor is calibrated.

The magnetic field sensor is configured to determine inclination of the radiation generation unit or the detection unit, respectively, based on the measurement of a magnetic field, for instance the earth magnetic field, which surrounds the sensor. The magnetic field sensor provides an absolute inclination of the radiation generation unit or the detection unit, respectively. However, the inclination information provided by the magnetic field sensor can be influenced by magnetic fields produced by devices (e.g. motors) in proximity of the sensor.

Advantageously, a combination of the inclination information of all three sensor types overcomes the limitations of each sensor type and allows for a particularly precise determination of the orientation and/or position of the radiation generation unit and the detection unit relative to each other.

In another preferred embodiment, the system comprises at least one transmitter configured to emit one or more signals and at least one receiver configured to receive the signals emitted by the transmitter, the transmitter or receiver being provided at the radiation generation unit, and the receiver or transmitter, respectively, being provided at the detection unit. Further, the processing unit is configured to determine a position of the radiation generation unit and the detection unit relative to each other by considering the signals received by the receiver and at least one of the first, second and third information captured by the accelerometer, gyroscope and magnetic field sensor, respectively, of the first sensor unit provided at the radiation generation unit, and at least one of the first, second and third information captured by the accelerometer, gyroscope and magnetic field sensor, respectively, of the second sensor unit provided at the detection unit. By this means, the determination of the position of the radiation generation unit and the detection unit relative to each other is particularly precise.

Preferably, the processing unit is configured to determine at least one distance between the at least one transmitter and the at least one emitter based on the one or more signals emitted and received. Further preferably, the processing unit is configured to determine the position of the radiation generation unit and the detection unit relative to each other, in particular a source-to-image distance (SID) between them, based on the at least one distance between the at least one transmitter and the at least one emitter by considering the orientation of the radiation generation unit and the detection unit relative to each other.

In particular, the distance between radiation generation unit and detection unit is determined by aligning coordinate systems of the at least one transmitter and the at least one receiver based on at least one of the first, second and third information provided by both the first and the second sensor unit and further considering the one or more signals emitted by the at least one transmitter and received by the at least one receiver. By this means, the determined distance between radiation generation unit and detection unit is particularly precise.

In another preferred embodiment, the system comprises at least three transmitters configured to emit one or more signals and at least three receivers configured to receive the signals emitted by the transmitters, wherein the at least three transmitters or at least three receivers are provided at the radiation generation unit, and the at least three receivers or at least three transmitters, respectively, are provided at the detection unit. The system further comprises a processing unit configured to determine, preferably at least six, distances between the at least three transmitters and the at least three receivers, and to determine an orientation of the radiation generation unit and the detection unit relative to each other by considering the determined distances between the at least three transmitters and at least three receivers. The processing unit is further configured to determine a position, in particular a distance, of the radiation generation unit and the detection unit relative to each other by considering the determined distances between the at least three transmitters and the at least three receivers and the orientation of the radiation generation unit and the detection unit relative to each other.

Preferably, the processing unit is configured to determine an orientation of the radiation generation unit and the detection unit relative to each other based on trilateration using the determined distances between the at least three transmitters and the at least three receivers, in particular by determining the orientation of the two planes in which the at least three transmitters and the at least three receivers are lying.

In other preferred embodiments, more than six, in particular nine or more than nine distances between three or more transmitters and three or more receivers may be determined by the processing unit, wherein not all of the determined distances are considered in the determination of the orientation of radiation generation unit and detection unit relative to each other. Further preferably, those determined distances between the receivers and the transmitters not considered may be used to cross-check the determined distances between the receivers and the transmitters considered in the determination of the orientation of radiation generation unit and detection unit relative to each other, or to cross-check said orientation.

In yet another preferred embodiment, the system comprises at least two transmitters configured to emit one or more signals and at least three receivers configured to receive the signals emitted by the transmitter or at least three transmitters configured to emit one or more signals and at least two receivers configured to receive the signals emitted by the transmitters, wherein the transmitters or receivers are provided at the radiation generation unit, and the receivers or transmitters are provided at the detection unit. The system further comprises a processing unit configured to determine at least six distances between the at least two transmitters and the at least three receivers or the at least three transmitters and at least two receivers, and to determine an orientation of the radiation generation unit and the detection unit relative to each other by considering the determined distances between the transmitters and the receivers and preferably the rotational degrees of freedom of the radiation generation unit and/or the rotational degrees of freedom of the detection unit. The processing unit is further configured to determine a position, in particular a distance, of the radiation generation unit and the detection unit relative to each other by considering the determined distances between the at least two transmitters and the at least three receivers or the at least three transmitters and the at least two receivers, and the orientation of the radiation generation unit and the detection unit relative to each other. This embodiment is particularly advantageous if the orientation of the radiation generation unit and detection unit relative to each other is limited by the rotational degrees of freedom of the radiation generation unit and/or the rotational degrees of freedom of the detection unit. For example, if the radiation generation unit may change its orientation relative to the detection unit only by rotation around two axes perpendicular to each other, and at least two transmitters or at least two receivers are provided at the radiation generation unit, the orientation of the radiation generation unit and the detection unit relative to each other may be determined reliably by considering the six distances between the at least two transmitters or receivers at the radiation generation unit and the at least three receivers or transmitters at the detection unit, respectively.

In another preferred embodiment of the invention, the one or more signals emitted by the at least one transmitter and received by the receiver are ultrasound signals or magnetic signals, in particular a magnetic flux, or electromagnetic signals, in particular light. This allows for a particular precise determination of the distance between the radiation generation unit and the detection unit.

In one embodiment, the receiver is provided at an edge of the detection unit so that the detection unit can be positioned beneath or behind the patient such that there is no part of the patient between the at least one transmitter and the receiver. This embodiment is particularly preferred if the one or more signals emitted by the at least one transmitter and received by the receiver are ultrasound or electromagnetic signals, in particular light, such that a reliable determination of the distance between the radiation generation unit and the detection unit is provided.

Preferably, the at least one transmitter is configured to generate a sequence of short pulses or a time varying signal, e.g. a sinusoidal signal. Further preferably, the processing unit is configured to analyze the pulse sequence or time varying signal received by the receiver, in particular by performing a run time measurement, i.e. a time-of-flight analysis, and/or a three-dimensional measurement of the signal amplitude, in particular of the magnetic flux, and/or a three-dimensional camera pattern recognition, in particular with light, allowing for a precise determination of the distance, in particular the SID, between the radiation generation unit and the detection unit.

In another preferred embodiment, the system comprises a third sensor unit configured to determine an inclination of the carriage with respect to the vertical and/or horizontal direction, wherein the processing unit is configured to control the drive unit to move the carriage based on the determined inclination of the carriage. This allows for a particular safe and reliable operation of the drive unit, in particular a precise positioning and/or orientation of the radiation generation unit and the detection unit relative to each other, when the mobile radiation image capturing system is positioned on a slope.

Preferably, the processing unit is configured to limit the maneuverability of the mobile carriage, for instance by preventing turns, rotations and/or sideway motion. By this means, an unintended movement of the carriage on slopes can be efficiently prevented, so that a reliable and particularly safe positioning and/or alignment of the radiation generation unit and the detection unit relative to each other is achieved.

Moreover, the processing unit is preferably configured to adjust the power of the drive unit, in particular a motor power, based on the determined inclination of the mobile carriage. For instance, if the system is moved up a ramp, the motor power is increased to maintain a constant movement of the system. Likewise, if the system is moved down a ramp, the motor power is decreased or inverted. This provides a particularly reliable and predictable movement of the carriage, in particular on slopes, whereby a particularly reliable and safe positioning and/or aligning of the radiation generation unit and the detection unit relative to each other is achieved.

Alternatively or additionally, the drive unit is provided with a kinetic energy recovery system (KERS), by means of which excess kinetic energy of the carriage, e.g. when moving down a slope, may be converted into electric energy and stored in an energy storage device, in particular a battery or battery module. Preferably, the processing unit is configured to activate and/or control the KERS based on the inclination of the carriage determined by the third sensor unit. In particular, the processing unit is configured to adjust the power of the drive unit according to the amount of kinetic energy converted by the KERS or vice versa.

Further preferably, the processing unit is configured to limit the maneuverability of the carriage and/or adjust the motor power of the drive unit if the carriage is tilted more than five degrees, i.e. if the floor exhibits a slope of more than approximately 10 %.

In another preferred embodiment, the radiation image capturing system comprises a handheld position tracker, at which a fourth sensor unit configured to capture fourth information regarding a position and/or orientation and/or movement of the handheld position tracker is provided, wherein the handheld position tracker is configured to be moved and/or tilted by a user, and the processing unit is configured to control the positioning unit to move the radiation generation unit based on the fourth information. Preferably, the handheld position tracker is configured to be grasped and/or grabbed by a hand or hands of the user. When the user performs a movement of his hand or hands while holding the handheld position tracker, the captured fourth information corresponds to the movement of the hand or hands, and the radiation generation unit preferably follows this movement. By this means, changing the position and/or orientation of the radiation generation unit, in particular relative to a patient and/or the detection unit, in particular bringing the radiation generation unit and the detection unit into an aligned position and/or orientation relative to each other, is accomplished in a particularly intuitive, fast and reliable way.

Preferably, the fourth sensor unit comprises one or more sensors configured to capture at least one of: an acceleration of the fourth sensor unit with respect to three spatial directions, an inclination of the fourth sensor unit with respect to three spatial directions, a magnetic field surrounding the fourth sensor unit with respect to three spatial directions. In particular, the fourth information corresponds to at least one of the acceleration of the fourth sensor unit, the inclination of the fourth sensor unit and the magnetic field surrounding the fourth sensor unit, and the processing unit is preferably configured to determine the movement of the handheld position tracker, at which the fourth sensor unit is provided, based on the fourth information.

Moreover, the processing unit is preferably configured to track, in particular to track changes of, the acceleration of the fourth sensor unit with respect to three spatial directions and/or the inclination of the fourth sensor unit with respect to three spatial directions and/or the magnetic field surrounding the fourth sensor unit with respect to three spatial directions over time. In this way, the movement, i.e. translation and/or tilt, of the handheld position tracker can be precisely determined.

In another preferred embodiment, the handheld position tracker comprises one or more tracking control elements for setting at least one tracking mode, and the processing unit is configured to control the positioning unit based on the fourth information and according to the tracking mode set by the one or more tracking control elements. Preferably, a first tracking control element activates, e.g. by pushing an activation button, the handheld position tracker, such that the processing unit controls the positioning unit to move, i.e. translate and/or tilt, the radiation generation unit based on the fourth information, as described above. Further preferably, the first tracking control element may also deactivate the handheld position tracker, e.g. by pushing the activation button again. Alternatively or additionally, the processing unit is configured to determine the orientation and/or position of the handheld position tracker, in particular relative to the orientation and/or position of the radiation generation unit, preferably by considering fourth information captured by the fourth sensor unit, provided at the handheld position tracker, and by considering first, second and third information captured by the first sensor unit, provided at the radiation generation unit. A second tracking control element activates a second tracking mode, in which the processing unit controls the positioning unit to move the radiation generation unit into a predefined position relative to the handheld position tracker. By this means, the position tracker may be used to mark a certain position, e.g. a body part of the patient, which is to be imaged, wherein the radiation generation unit is positioned automatically in an orientation and/or position relative to the marked position such that high quality radiation images may be reliably captured.

Further advantages, features and examples of the present invention will be apparent from the following description of following figures:

Fig. 1   shows a front view of an example of a radiation image capturing system at a patient's bedside;

Fig. 2   shows a side view of another example of a radiation image capturing system;

Fig. 3   shows a schematic representation of a first example of a processing unit and components connected to the processing unit;

Fig. 4   shows a side view of another example of a radiation image capturing system;

Fig. 5   A to D shows top views of another example of a radiation image capturing system in order to illustrate possible movements of the system; and

Fig. 6   Shows a schematic representation of a second example of a processing unit and components connected to the processing unit.

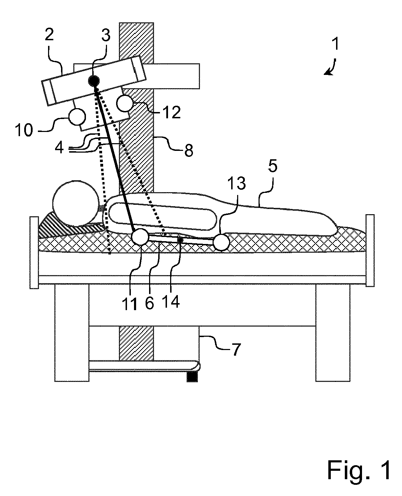

Figure 1 shows a front view of an example of a radiation image capturing system 1 which is located at a patient's bedside. A radiation generation unit 2 comprises a radiation source 3, also referred to as X-ray tube, which is configured to generate X-ray radiation 4. Below the radiation generation unit 2, the patient 5 to be examined lies on a bed. X-ray radiation 4 transmits through the patient 5 and impinges on a detection unit 6, which is configured to detect the radiation, e.g. by converting it into electrical signals by means of a solid-state detector, by storing it in a storage phosphor sheet or by recording it on a photographic film. The detection unit 6 is portable, such that it can be easily positioned into a desired orientation and/or position beneath or behind the patient 5.

Figure 1 shows a front view of an example of a radiation image capturing system 1 which is located at a patient's bedside. A radiation generation unit 2 comprises a radiation source 3, also referred to as X-ray tube, which is configured to generate X-ray radiation 4. Below the radiation generation unit 2, the patient 5 to be examined lies on a bed. X-ray radiation 4 transmits through the patient 5 and impinges on a detection unit 6, which is configured to detect the radiation, e.g. by converting it into electrical signals by means of a solid-state detector, by storing it in a storage phosphor sheet or by recording it on a photographic film. The detection unit 6 is portable, such that it can be easily positioned into a desired orientation and/or position beneath or behind the patient 5.

In the example shown in Figure 1, the radiation generation unit 2 and the detection unit 6 are not in an aligned orientation and/or position relative to each other, because the X-ray radiation 4, in particular the central beam, generated by the radiation source 3 does not impinge orthogonally on the detection unit 2 and illuminates only a part of the sensitive area of the detection unit 6. Further, the X-ray radiation 4, in particular the X-ray radiation cone, is not centered on the detection unit 2, i.e. the center beam of the X-ray radiation 4 does not coincide with a predetermined position 14 at, i.e. the center of, the detection unit 2. For the sake of completeness it is pointed out that, in some X-ray exams, an orthogonal center beam of the X-ray radiation 4 is not necessarily required. However, the center beam should correspond more or less with the center of the detection unit 2.

In order to bring the radiation generation unit 2 and the detection unit 6 in an aligned position and/or orientation relative to each other, the radiation generation unit 2 is movably mounted on a carriage 7, in particular on a column 8 of the carriage 7, such that it can be translated and/or rotated relative to the carriage 7 and/or the column 8 until the radiation generation unit 2 and the detection unit 6 are properly aligned relative to each other, e.g. such that the center beam of the X-ray radiation 4 impinges on the detection unit 6 with a pre-specified angle of incidence, e.g. orthogonally, and/or the beam cone of the X-ray radiation 4 illuminates a pre-specified area of the detection unit 6. Preferably, the radiation generation unit 2 and the detection unit 6 can be aligned such that essentially all of the X-ray radiation 4 emitted by the radiation generation unit 2 is detected by the detection unit 6.

The radiation image capturing system 1 comprises a first sensor unit 10, which is provided at the radiation generation unit 2, in particular mounted at a housing of the radiation generation unit 2. The first sensor unit 10 is configured to provide information on the orientation and/or the position of the radiation generation unit 2, in particular on its inclination.

Moreover, a second sensor unit 11 is provided at the detection unit 6. The second sensor unit 11 is configured to provide information on the orientation and/or the position of the detection unit 6, in particular on its inclination. By combining the information provided by the first sensor unit 10 and the information provided by the second sensor unit 11, an orientation and/or a position of the radiation generation unit 2 and the detection unit 6 relative to each other can be determined as explained further below.

Preferably, the system 1 further comprises a transmitter 12, which is preferably provided at the radiation generation unit 2, in particular mounted at the housing of the radiation generation unit 2, and is configured to emit one or more signals, e.g. magnetic signals, electromagnetic signals, in particular light, and/or ultrasound signals. A receiver 13, which is provided at the detection unit 6, is configured to receive the one or more signals emitted by the transmitter 12. In an alternative embodiment (not shown), the transmitter 12 may be provided at the detection unit 6 and the receiver 13 may be provided at the radiation generation unit 2. Based on the one or more signals received by the receiver 13, a position and/or a distance of the radiation generation unit 2 and the detection unit 6 relative to each other, in particular the source-to-image distance (SID) of the radiation source 3 to a predefined position 14 at the detection unit 6, can be determined as explained further below.

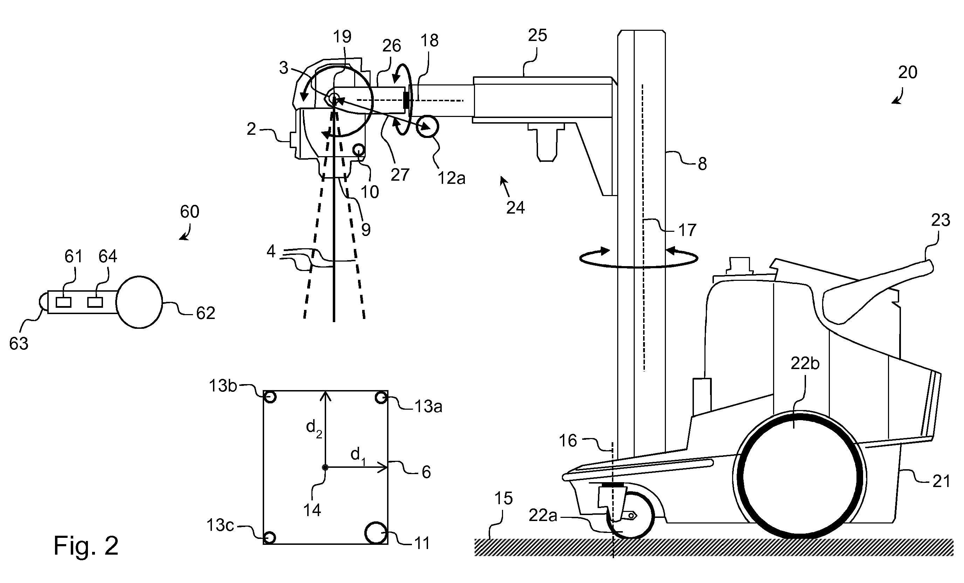

Figure 2 shows a side view of another example of a radiation image capturing system 20 comprising a mobile carriage 21 which is equipped with wheels 22a and 22b by which the carriage 21 can be moved across the floor 15. Preferably, first wheels 22a are designed as caster wheels which are configured to be rotated around a vertical axis 16 in order to provide high maneuverability of the carriage 21. Alternatively or additionally, second wheels 22b are designed as drive wheels 22b which are preferably coupled to a driving means, e.g. a motor, which is configured to drive the second wheels 22b.

Preferably, the carriage 21 comprises a handle 23 which can be actuated and/or grasped by a user to move, in particular to push and/or maneuver, the carriage 21.

On the carriage 21 a vertical column 8 is mounted, which is configured to be rotated around a first rotation axis 17, as indicated by a curved arrow around the column 8. On the column 8 a tube arm 24 is mounted at the distal end of which a radiation generation unit 2 is movably mounted.

The tube arm 24 comprises a first arm element 25 and a second arm element 26, wherein the second arm element 26 is rotatably mounted on the first arm element 25 such that it can be rotated around a second rotation axis 18 as indicated by a curved arrow around the second arm element 26. Further, the radiation generation unit 2 is rotatably mounted on the second arm element 26 such that it can be rotated around a third rotation axis 19 which is perpendicular to the image plane of Figure 2. The movement of the radiation generation unit 2 upon a rotation around the third rotation axis 19 is indicated by a curved arrow around the third rotation axis 19. As a result, the radiation generation unit 2 is movably mounted on the first arm element 25 by a gimbal joint, i.e. it can be rotated such that an aperture 9 of the X-ray radiation 4 generated by the radiation generation unit 2 can be directed in any desired direction.

In a preferred embodiment (not shown), the first arm element 25 movably mounted on the column 8 such that it can be translated upwards and/or downwards along the column 8 in order to adjust the height of the radiation generation unit 2 relative to the floor 15. Alternatively or additionally, the first arm element 25 is a telescopic arm element, i.e. a first part of the first arm element 25 can be retracted into a second part of the first arm element 25, such that the distance between the radiation generation unit 2 and the column 8, i.e. the length of the tube arm 24, can be adjusted.

By means of the embodiments described above, preferably in combination with the maneuverability of the mobile carriage 21, the user has a number of degrees of freedom to adjust the orientation and/or position of the radiation generation unit 2 relative to a patient and/or a detection unit 6. As a result, the radiation generation unit 2 and the detection unit 6 may be easily and reliably brought into a desired and/or an aligned orientation and/or position relative to each other.

Like in the example shown in Figure 1, a first sensor unit 10, which determines information on an orientation and/or a position of the radiation generation unit 2, is mounted at the radiation generation unit 2, whereas a first distance sensor 12a, which is configured as a transmitter, is provided at the first arm element 25 in a fixed or predetermined distance 27 to the radiation source 3. This arrangement advantageously prevents or at least minimizes an influence on the inclination or position detection in the first sensor unit 10 by signals, e.g. magnetic, electromagnetic or ultrasound signals, emitted by the first distance sensor 12a.

In the embodiment shown in Figure 2 three second distance sensors 13a, 13b, 13c, which are configured as receivers, are provided at three corners of the detection unit 6, in particular at positions [d1, d2, 0], [-d1, d2, 0], [-d1, -d2, 0] in a coordinate system having its origin in a predetermined position 14, e.g. a center position, at the detection unit 6 and the z-axis perpendicular to the image plane. Each of the three second distance sensors 13a, 13b, 13c is configured to receive the one or more signals emitted by the first distance sensor 12a.

In an alternative embodiment, each of the three second distance sensors 13a, 13b, 13c is configured to emit one or more signals, e.g. magnetic, electromagnetic or ultrasound signals, and the first distance sensor 12a is configured to receive the one or more emitted signals. Based on the one or more signals received by the second distance sensors 13a, 13b, 13c, the distances between the first distance sensor 12a and each of the three second distance sensors 13a, 13b, 13c can be precisely determined.

Preferably, the source-to-image distance (SID) between the radiation source 3 and the predetermined position 14 at the detection unit 6 is determined based on the determined distances between the first distance sensor 12a and each of the three second distance sensors 13a, 13b, 13c and the fixed or predetermined distance 27 between the first distance sensor 12a and the radiation source 3.

Preferably, the SID between the radiation source 3 and the predefined position 14 at the detection unit 6 is determined not only by considering the determined distances between the second distance sensors 13a, 13b, 13c and the first distance sensor 12a and/or the distance 27 between the first distance sensor 12a and the radiation source 3, but also by considering information on an orientation of the detection unit 6 and/or the radiation generation unit 2, which is provided by the second sensor unit 11 or first sensor unit 10, respectively, which is described in detail further below.

Preferably, the distance sensors 12a, 13a, 13b, 13c are designed as magnetic coils, in particular coil triads each comprising three coils having winding axes perpendicular to each other, such that by applying an alternating current to the first distance sensor 12a, which acts as a transmitter, magnetic flux signals are emitted which may be received by the second distance sensors 13a, 13b, 13c, which act as receivers, by measuring the respective voltage and/or current induced in the coils. for example, the distances between the first distance sensor 12a and the three second distance sensors 13a, 13b, 13c can be determined by summing up the squares of the induced currents/voltages. Alternatively or additionally, the distances between the first distance sensor 12a and the three distance sensors 13a, 13b, 13c can be determined based on the induced currents/voltages and a predetermined correction or calibration factor taking into account the non-ideality of the system.

Alternatively, the distance sensors 12a, 13a, 13b, 13c are designed as ultrasound transmitters and receivers, respectively, whereby the distance between the first distance sensor 12a and the three second distance sensors 13a, 13b, 13c can be calculated by a run time measurement of one or more ultrasound signals emitted by the first distance sensor 12a and received by the three second distance sensors 13a, 13b, 13c.

In another embodiment, the radiation image capturing system 1 as shown in Figure 1 and/or the mobile radiation image capturing system 20 as shown in Figure 2 comprises a handheld position tracker 60 configured to capture movement instructions, based on which the radiation generation unit 2 is oriented and/or positioned, in particular in an aligned position relative to the detection unit 6. The handheld position tracker 60 is designed to be grasped and/or grabbed by the user. Upon activation of the handheld position tracker 60, e.g. by pushing an activation button 61, a fourth sensor unit 62 provided at the handheld position tracker 60 captures fourth information regarding a position and/or inclination and/or movement of the handheld position tracker 60, i.e. position and/or inclination and/or movement information. As described in more detail further below, the radiation image capturing system 1 and/or the mobile radiation image capturing system 20 is configured to receive the fourth information transmitted by a tracking transmitter 63 provided at the handheld position tracker 60 and to position, i.e. to translate and/or to tilt, the radiation generation unit 2 according to the captured fourth information or information derived therefrom. The tracking transmitter 63 is preferably part of a wireless communication system, e.g. bluetooth or a wireless LAN.

Preferably, the radiation generation unit 2 is moved, i.e. translated and/or tilt, in accordance with the movement of the handheld position tracker 60. That is, the radiation generation unit 2 follows the motion of the user's hand(s) holding the handheld position tracker 60. In this way, the radiation generation unit 2 can be brought into an aligned orientation and/or position relative to the detection unit 6 in a fast, intuitive and reliable way.

Preferably, the fourth sensor unit 62 comprises sensors configured to capture information regarding acceleration and/or inclination of the fourth sensor unit 62 relative to three spatial directions and/or a magnetic field surrounding the fourth sensor unit 62 relative to the three spatial directions. By tracking the acceleration and/or the inclination and/or the magnetic field signals, in particular over time, a processing unit may determine a position and/or inclination and/or movement of the handheld position tracker 60. In another preferred embodiment, the handheld position tracker 60 is configured to be activated by a user, i.e. by pushing the activation button 61, and to be placed onto a patient, in particular onto a body part of the patient to be imaged. By pushing an alignment button 64 on the handheld position tracker 60, the radiation generation unit 2 is centered on the position marked by the handheld position tracker 60 automatically, i.e. the radiation generation unit 2 and the detection unit 6 are brought into a predetermined orientation and/or position relative to each other. Alternatively or additionally, the alignment button 64 is provided at the carriage 21 and/or at the radiation generation unit 2 (not shown). Basically, the alignment button 64 may not necessarily be a button in the narrower sense, but also any another kind of a control element, e.g. a lever, knob or a touch sensitive screen.

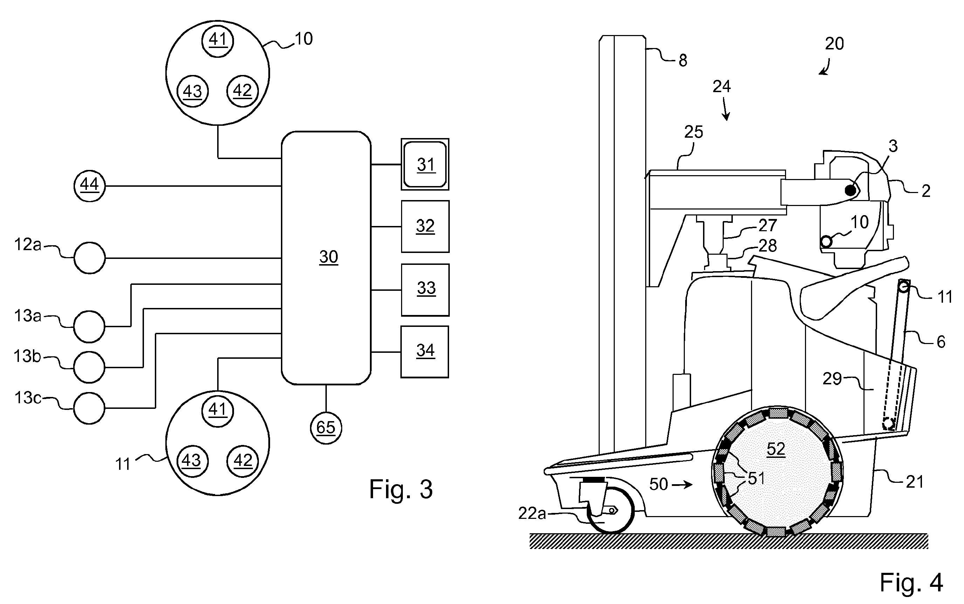

Figure 3 shows a schematic representation of an example of a processing unit 30, to which components of the radiation image capturing system are connected, in particular the first sensor unit 10, the second sensor unit 11, the first distance sensor 12a, three second distance sensors 13a, 13b, 13c, an output unit 31, a control element 32, and a drive unit 33. The processing unit 30 is configured to obtain signals and/or information from the sensors and/or sensor units, to process the obtained signals and/or information, to output the processed signals and/or information and/or to further use the obtained and/or processed signals and/or information for controlling the system or components thereof.

Preferably, the first sensor unit 10 and the second sensor unit 11 each comprise an acceleration sensor 41 configured to capture first information, a gyroscope sensor 42 configured to capture second information, and a magnetic field sensor 43 configured to capture third information.

The first information relates to an acceleration of the acceleration sensor 41 with respect to three spatial directions, in particular relative to the gravity vector. If the acceleration sensor 41 is not accelerated by movement, an absolute orientation of the acceleration sensor 41 contained in the first sensor unit 10 and/or the second sensor unit 11 can be determined by the processing unit 30.

The second information relates to an inclination of the gyroscope sensor 42 relative to a predefined axis with respect to three spatial directions, such that a relative orientation of the gyroscope sensor 42 contained in the first sensor unit 10 and/or the second sensor unit 11 can be determined by the processing unit 30.

The third information relates to a magnetic field, in particular an orientation of a magnetic field, in particular the earth's magnetic field, which surrounds the magnetic field sensor 43 with respect to three spatial directions. If the magnetic field is not distorted, e.g. by magnetic fields generated by devices in the vicinity such as motors, the absolute orientation of the magnetic field sensor 43 contained in the first sensor unit 10 and/or the second sensor unit 11 can be determined by the processing unit 30.

Because both of the first sensor unit 10 and the second sensor unit 11 comprise three sources of inclination information each with respect to three spatial directions or spatial axis, the first sensor unit 10 and the second sensor unit 10 may be regarded as nine-dimensional (9D) or 9-axis inclination sensors.

Preferably, the processing unit 30 is configured to determine an orientation of the first sensor unit 10 and/or the second sensor unit 11 and/or an orientation of the radiation generation unit 2 and the detection unit 6 relative to each other based on the first, second and third information. The determined orientation is particularly precise and reliable, because it is based on information captured by three different sensor types. If an inclination measurement by one of the three sensor types is adversely affected, e.g. in case that the acceleration sensor 41 is accelerated by movement or an interfering magnetic field is close to the magnetic field sensor 43, still two of the three sensor types provide correct information or provide necessary information to compensate for effects adversely influencing the inclination measurement of the one sensor.

In a preferred embodiment, the processing unit 30 is configured to further determine the position of the first sensor unit 10 and/or the second sensor unit 11 and/or the radiation generation unit 2 and the detection unit 6, in particular relative to each other, by tracking the changes of first, second and third information provided by the first sensor unit 10 and/or the second sensor unit 11. For example, by tracking the acceleration, in particular the direction of the acceleration, of the acceleration sensor 41 over time, the distance covered by the acceleration sensor 41 and thereby its spatial position can be determined.

Alternatively or additionally, the processing unit 13 is configured to determine a position of the radiation generation unit 2 and the detection unit 6 relative to each other, in particular a distance between them, based on the one or more signals emitted by the first distance sensor 12a and received by the three second distance sensors 13a, 13b, 13c, preferably by also considering the orientation of the radiation generation unit 2 and the detection unit 6 relative to each other which has been determined based on the first, second and third information.

Preferably, the processing unit 30 is configured to determine the distance between the radiation source 3 and the predefined position 14 at the detection unit 6 (see Figures 1 and 2) based on position coordinates $[X_p, Y_p, Z_p]$ of the predefined position 14 in a coordinate system having its origin at the first distance sensor 12a, wherein the position coordinates $[X_p, Y_p, Z_p]$ of the predefined position 14, which corresponds to the origin of a coordinate system of the detection unit 6, are obtained by the following transformation:

$$\begin{bmatrix} x_p \\ y_p \\ z_p \end{bmatrix} = \vec{\vec{\rho}}^{-1} \cdot \begin{bmatrix} M_1 \\ M_2 \\ M_3 \end{bmatrix}, \qquad (1)$$

wherein p is a transformation matrix

$$\vec{\vec{\rho}} = \begin{bmatrix} -1 & \rho_1 & \rho_2 \\ \rho_4 & \rho_5 & \rho_6 \\ \rho_1 & 1 & \rho_3 \end{bmatrix}$$

with components

$$\rho_1 := r_{11} \cdot r_{12} + r_{21} \cdot r_{22} + r_{31} \cdot r_{32};$$

$$\rho_2 := r_{11} \cdot r_{13} + r_{21} \cdot r_{23} + r_{31} \cdot r_{33};$$

$$\rho_3 := r_{12} \cdot r_{13} + r_{22} \cdot r_{23} + r_{32} \cdot r_{33};$$

$$\rho_4 := \left( d_1 + d_2 \rho_1 \right);$$

$$\rho_5 := \left( d_2 + d_1 \rho_1 \right);$$

$$\rho_6 := \left( d_1 \rho_2 + d_2 \rho_3 \right),$$

wherein the $r_{ij}$ with i=1,2,3 and j=1,2,3 are the components of a transformation (rotation) matrix

$$\ddot{R} = \begin{bmatrix} r_{11} & r_{12} & r_{13} \\ r_{21} & r_{22} & r_{23} \\ r_{31} & r_{32} & r_{33} \end{bmatrix}$$

for aligning the coordinate system of the sensor unit 6 with the coordinate system of the first distance sensor 12a, and $d_1$ and $d_2$ are components of the position vectors $s^i$ of the three second distance sensors 13a, 13b, 13c in the coordinate system of the detection unit 6 (see Figure 2):

$$\vec{s}^1 = \begin{bmatrix} d_1 \\ d_2 \\ 0 \end{bmatrix}; \vec{s}^2 = \begin{bmatrix} -d_1 \\ d_2 \\ 0 \end{bmatrix}; \vec{s}^3 = \begin{bmatrix} -d_1 \\ -d_2 \\ 0 \end{bmatrix}.$$

The components of the transformation (rotation) matrix R can be determined by considering the first, second and third information provided by the second sensor unit 11 and, if the first distance sensor 12a is mounted to the radiation generation unit 2, by also considering the first, second and third information provided by the first sensor unit 10, such that the orientation of the radiation generation unit 2 and the detection unit 6 relative to each other can be determined.

If the first distance sensor 12a is mounted to the tube arm 24, as shown in Figure 2, its coordinate system is fixed such that first, second and third information provided by the first sensor unit 10 does not necessarily have to be considered. Instead, the distance 27 between the radiation source 3 and the first distance sensor 12a is preferably added to the vector coordinates $[X_p, Y_p, Z_p]$ of the origin of the coordinate system of the detection unit 6.

The coordinates $M_i$ with i=1,2,3 in equation (1) are defined as

$$M_1 := \frac{D_1^2 - D_2^2}{4 d_1} - d_2;$$

$$M_2 := \frac{D_3^2 - D_1^2}{4};$$

$$M_3 := \frac{D_3^2 - D_2^2}{4d_2} - 4d_1\rho_1;$$

wherein $D_i$ with i=1,2,3 are the measured distances between the first distance sensor 12a and the three second distance sensors 13a, 13b, 13c, for instance by run time measurements of one or more ultrasound signals or summing up the squares of the current/voltage which is induced by one or more magnetic flux signals.

The inverse of p can be calculated by

$$\vec{\rho}^{-1} = \frac{adj(\vec{\rho})}{\det(\vec{\rho})};$$

wherein

$$\det(\vec{\rho}) = \rho_1^2\rho_6 + \rho_4\rho_4 + \rho_6 - \rho_1\rho_3\rho_4 - \rho_1\rho_5\rho_2 - \rho_5\rho_3$$

and

$$adj(\vec{\rho}) = \begin{bmatrix} [\rho_3\rho_5 - \rho_6] & [\rho_2 - \rho_1\rho_3] & [\rho_1\rho_6 - \rho_2\rho_5] \\ [\rho_1\rho_6 - \rho_3\rho_4] & [-\rho_3 - \rho_1\rho_2] & [\rho_6 + \rho_2\rho_4] \\ [\rho_4 + \rho_1\rho_5] & [\rho_1^2 + 1] & [-\rho_5 - \rho_1\rho_4] \end{bmatrix}$$

The processing unit 30 is further configured to output positioning information, i.e. information regarding the orientation and/or position of the radiation generation unit 2 and the detection unit 6, in particular relative to each other, to the user via an output unit 31. The output unit 31 can be designed as a display and/or a speaker such that the positioning information may be provided visually and/or acoustically, respectively.

In a preferred embodiment, the display may show a graphical representation of the radiation generation unit 2 and the detection unit 6 and their relative orientation and/or position such that the user may recognize how to move, i.e. translate and/or rotate, the radiation generation unit 2 and/or the detection unit 6 in order to bring them into an aligned position relative to each other. Alternatively or additionally, the display shows movement instructions on how to bring the radiation generation unit 2 and the detection unit 6 into an aligned position relative to each other by graphically indicating the necessary movement, e.g. by arrows.

In another preferred embodiment, the speaker outputs the positioning information in spoken words addressing the user, who may position the radiation generation unit 2 and/or the detection unit 6 accordingly. Additionally or alternatively, the speaker outputs an audio signal indicating an alignment of the radiation generation unit 2 and the detection unit 6 relative to each other. Preferably, the speaker outputs an audio signal or a series of audio signals whose pitch and/or repetition frequency increases or decreases the closer the radiation generation unit 2 and/or the detection unit 6 come to a preferred alignment relative to each other.

Preferably, the output unit 31 is provided in the vicinity of the handle 23 of the carriage 21 (see Figure 2), such that the user can access the positioning information output via the output unit 31 while standing behind the carriage 21. Additionally or alternatively, the output unit 31 is provided at the radiation generation unit 2, i.e. at the end of the tube arm 24 opposite to the column 8, such that the user can access the positioning information during an adjustment of the the radiation generation unit 2 relative to a patient and/or the detection unit 6.

The processing unit 30 is further configured to receive movement instructions regarding a desired change in orientation and/or position of the radiation generation unit 2 and the detection unit 6, in particular relative to each other. Preferably, the movement instructions are input by a user via a control element 32. The control element 32 may comprise, e.g., a control stick and/or buttons and/or levers and/or a touch-sensitive display, which is or are configured to be operated by the user according to the desired movement of the mobile carriage 21 and/or the radiation generation unit 2. Alternatively or additionally, the control element 32 comprises a microphone configured to record spoken movement instructions of the user, in particular movement commands for moving the carriage 21, which are processed, i.e. interpreted, by the processing unit 30.

Preferably, the control element 32 is provided in the vicinity of the handle 23, such that the user may input movement instructions when standing behind the carriage 21. Alternatively or additionally, the control element 32 is provided at the radiation generation unit 2, i.e. at the end of the tube arm 24 opposite of the column 8. This allows for altering the orientation of and/or position of the radiation generation unit 2, in particular relative to the detection unit 6, while operating the radiation generation unit 2.

Preferably, the processing unit 30 processes the movement instructions and controls a drive unit 33 accordingly. The drive unit 33 may be a motor, in particular an electric motor, connected to the drive wheels 22b of the mobile carriage 21 (see Figure 2), such that upon an input of movement instructions by the user, the mobile carriage 21 moves, by translation and/or rotation, across the floor 15.

Alternatively or additionally, the processing unit 30 is configured to control a positioning unit 34 which is configured to move, i.e. translate and/or rotate, the radiation generation unit 2 relative to the carriage 21 and/or the detection unit 2. In particular, a rotation of the column 8 around the first rotation axis 17, a rotation of the second arm element 26 around the second rotation axis 18 and/or a rotation of the radiation generation unit 2 around the third rotation axis 19 may be controlled by the user by inputting according movement instructions via the control element 32. Further, adapting a height of the radiation generation unit 2 relative to the floor 15 by translating the first arm element 25 along the column 8 and/or adjusting a distance between the radiation generation unit 2 and the column 8 by retracting or extending the first part of the first arm element 25 into or out of the second part of the first arm element 25 may be controlled.

As shown in Figure 3, a third sensor unit 44 may be provided which is configured to provide inclination information on an inclination of the mobile carriage 21. Preferably, this inclination information is also considered by the processing unit 30 when determining the orientation and/or position of the radiation generation unit 2 and the detection unit 6 relative to each other. Further, the information on the inclination of the carriage 21 may be used by the processing unit 30 when controlling the drive unit 33 to move the carriage 21. For instance, the slope of the floor 15 can advantageously be considered in the movement of the carriage 21.

In a preferred embodiment, the processing unit 30 adjusts the power of the drive unit 33 based on the information on the inclination of the mobile carriage 21 provided by the third sensor unit 44 such that the velocity of the carriage 21 is substantially constant regardless whether the carriage 21 moves up a slope or down a slope of the floor 15. Further, the processing unit 30 is preferably configured to control the movement of the carriage 21 such that sharp turns of the carriage 21 are avoided if the carriage 21 is located on a floor 15 having a slope of 5°, that is 9 %, or larger.

In another embodiment, the radiation image capturing system 20 comprises a tracking receiver 65 which is configured to receive fourth information captured by the handheld position tracker 60 and wirelessly transmitted by the tracking transmitter 63 (see Figure 2), e.g via bluetooth or a wireless LAN. In particular, the tracking receiver 65 receives signals emitted by the tracking transmitter 63 comprising information about the translation and/or tilt, in particular the direction of translation and/or tilt, of the handheld position tracker 60. The processing unit 30 is configured to control the drive unit 33 based on the movement information, i.e. to position the carriage 21 such that the radiation generation unit 2 follows the movement performed by the handheld position tracker 60. Alternatively or additionally, the processing unit 30 is configured to control the positioning unit 34 which is configured to rotate the column 8 and/or rotate the second arm element 26 and/or rotate the radiation generation unit 2 and/or extend and/or retract the first arm element 25 and/or translate the tube arm 24 along the column 8, such that the radiation generation unit 2 moves in accordance with the handheld position tracker 60. By this means, the radiation generation unit 2 follows the movement of the handheld position tracker 60, i.e. the hand(s) of the user holding the handheld position tracking 60.

In another preferred embodiment, the tracking transmitter 63 is configured to transmit information regarding acceleration and/or inclination of the fourth sensor unit 62 (shown in Figure 2) relative to three spatial directions and/or a magnetic field surrounding the fourth sensor unit 62 relative to the three spatial directions. The processing unit 30 controls the drive unit 33 or further drive units (not shown) to move, i.e. translate and/or tilt, the radiation generation unit 2 in accordance with the handheld position tracker 60 based on the information received by the tracking receiver 65.

Figure 4 shows another example of a radiation image capturing system 20, wherein the radiation generation unit 2 is retained in a first reference position and the detection unit 6 is retained in a second reference position. The first reference position corresponds to a position of the radiation generation unit 2 and the tube arm 24, in which the radiation generation unit 2 is arranged essentially parallel to the column 8 such that a straight line running through the radiation source 3 and the center of the aperture 9 is essentially parallel to the column 8 and in which the tube arm 24 is retracted, i.e. where the first part of the first arm element 25 is retracted into the second part of the first arm element 25. Further, in the first reference position, the tube arm 24 is lowered along the column 8 such that a retaining element 27, which is provided at the tube arm 24, rests on and/or is releasably coupled with a support element 28, which is provided at the carriage 21. In the latter case any movement of the radiation generation unit 2 is reliably prevented. In this way, an initial or reference position and/or orientation of the radiation generation unit 2 is defined.

The detection unit 6 is retained in the second reference position by means of a second retaining element 29, e.g. a receptacle into which the detection unit 6 may be inserted such that any movement of the detection unit 6 is reliably prevented. Preferably, the receptacle is a slot into which the detection unit 6 may be fully or partially inserted. In this way, an initial or reference position and/or orientation of the detection unit 6 is defined.

When the radiation generation unit 2 and the detection unit 6 are in their first and second reference position, respectively, their orientation and/or position relative to each other is defined such that the sensors 41, 42, 43 of the first sensor unit 10 and/or the sensors 41, 42, 43 of the second sensor unit 11 can be calibrated (see Figure 3). This may be done, for example, by capturing calibration values, i.e. first, second and third information of each of the first sensor unit 10 and the second sensor unit 11, and saving it in a memory, in particular in a ring buffer. Preferably, the calibration defines the axis relative to which the gyroscope sensor 42 provides information on a relative inclination. Thus, if the calibration has been performed and the radiation generation unit 2 and/or the detection unit 6 is/are removed from their respective reference position, the processing unit 30 may reliably determine their current orientation and/or position relative to each other based on current first, second and third information provided by the first sensor unit 10 and the second sensor unit 11 and on calibration values read from the ring buffer.

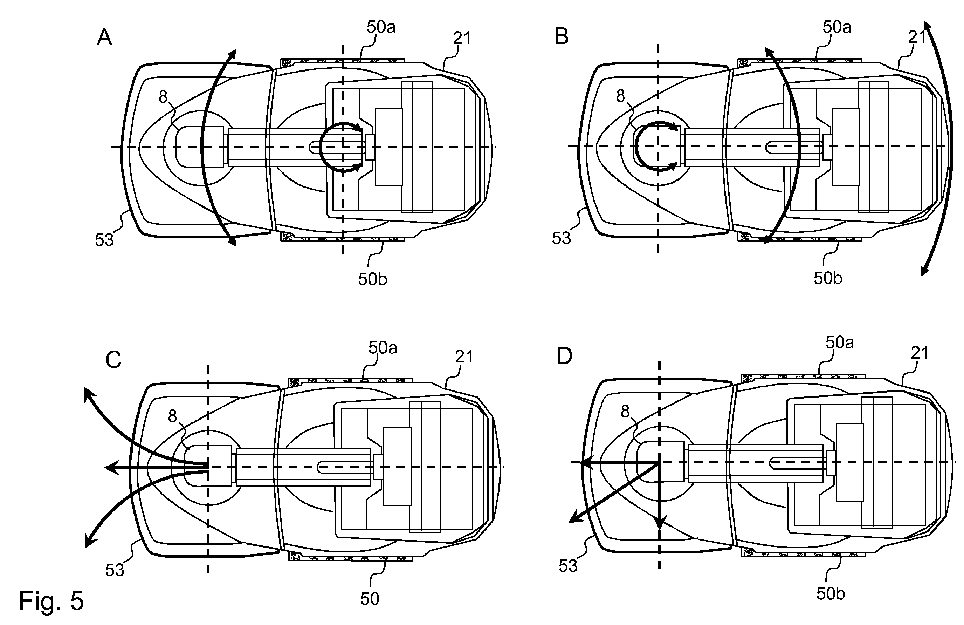

The mobile carriage 21 shown in Figure 4 comprises one or more omnidirectional wheels 50 by which the maneuverability of the carriage 21 is further enhanced considerably. Preferably, the wheels 50 are configured to allow for sideways and/or diagonal movement of the carriage 21, as exemplarily illustrated in Figure 5 and described further below. The one or more omnidirectional wheels 50 preferably comprise several rollers 51 arranged at the circumference of a center wheel 52. Each of the rollers 51 is configured to rotate around a respective roller axis (not shown), wherein each of the roller axis is perpendicular to the axis (not shown) of the center wheel 52. In particular, each of the roller axis is essentially tangential to the center wheel 52.

Figure 5 shows examples of movements of a mobile radiation image capturing system 20 in a top view, wherein the carriage 21 is depicted schematically. By providing the mobile carriage 21 with at least one caster wheel 22a (see Figure 4) and two omnidirectional wheels 50a, 50b, the carriage 21 may be rotated around different axes as indicated in Figure 5A and Figure 5B by curved arrows. Alternatively or additionally, the omnidirectional wheels 50a, 50b may be configured as mecanum wheels (not shown). Further alternatively or additionally, the omnidirectional wheels may be configured as omni wheels with two drives, wherein each of the omni wheels consists of a larger first wheel which is configured for driving a forward movement by rotation around a first main axis and a second smaller wheel which is configured for driving a sideward movement or a rotation of the carriage by rotation around a second main axis, and wherein the first larger wheel and the second smaller wheel are arranged one after another, such that the first main axis and the second main axis are perpendicular to one another and parallel to the floor, respectively (not shown).In Figure 5A, the center of the rotation lies within the center of mass or in the vicinity thereof of the carriage 21, which is indicated by the intersection point of the two dashed lines. In this case, the carriage 21 essentially rotates in place. In Figure 5B, the center of the rotation lies within the column 8, which is again indicated by the intersection point of the two dashed lines.

Moreover, the carriage 21 may move in different directions parallel to the floor 15 as indicated in Figure 5C and Figure 5D by the curved or straight arrows. In Figure 5C, if the carriage 21 follows the direction indicated by the curved arrows, the orientation of the carriage 21, i.e. the direction to which the front end 53 of the carriage 21 faces, changes. In Figure 5D, if the carriage 21 follows the direction of the arrows, the orientation of the carriage 21 does not change, i.e. the carriage 21 may perform a diagonal or sideways motion.

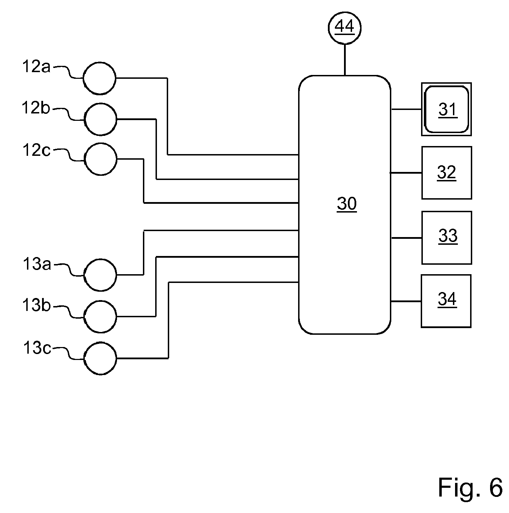

Figure 6 shows a schematic representation of a second example of the processing unit 30, to which several components of the radiation image capturing system are connected, in particular three first distance sensors 12a, 12b, 12c, three second distance sensors 13a, 13b, 13c, a third sensor unit 44, an output unit 31, a control element 32, a drive unit 33, and a movement unit 34. The processing unit 30 is configured to obtain signals and/or information from the sensors and/or sensor units, to process the obtained signals and/or information, to output the processed signals and/or information and/or to further use the obtained and/or processed signals and/or information for controlling the system or components thereof. Moreover, the processing unit 30 is configured to determine the distances between the three first distance sensors 12a, 12b, 12c provided at the radiation generation unit 2 (see Figures 1 and 2) and the three second distance sensors 13a, 13b, 13c provided at the detection unit 6 (see Figures 1 and 2) based on at least one signal emitted or received by the three first distance sensors 12a, 12b, 12c and received or emitted, respectively, by the three second distance sensors 13a, 13b, 13c. Preferably, the processing unit 30 determines the distance between each of the three first distance sensors 12a, 12b, 12c and each of the three second distance sensors 13a, 13b, 13c, respectively, such that in total nine distances can be determined.

Further, the processing unit 30 is configured to determine the orientation of the radiation generation unit 2 and the detection unit 6 relative to each other based on the determined distances between the three first distance sensors 12a, 12b, 12c and the three second distance sensors 13a, 13b, 13c, in particular by determining the positions of the several distance sensors relative to each other by trilateration based on the determined distances and calculating the relative orientation of the two planes in which the three first distance sensors 12a, 12b, 12c and the three second distance sensors 13a, 13b, 13c are lying.

Preferably, the position, in particular the distance, of the radiation generation unit 2 and the detection unit 6 relative to each other is determined by the processing unit 30 as described above with reference to Figure 3, wherein the components of the transformation (rotation) matrix R are determined by considering the determined distances between the three first distance sensors 12a, 12b, 12c and the three second distance sensors 13a, 13b, 13c.

Although the three first distance sensors 12a, 12b, 12c and the three second distance sensors 13a, 13b, 13c allow for the determination of nine distances between them, in other embodiments, where more than three first distance sensors 12a, 12b, 12c and more than three second distance sensors 13a, 13b, 13c are provided, more than nine distances may be determined. Preferably, the resulting redundancy is used for cross-checking the determined distances between the several distance sensors, and in particular the determined position and/or distance of the radiation generation unit 2 and the detection unit 6 relative to each other. This is particularly advantageous if the at least one signal emitted and received by the several distance sensors is affected by nearby (electro)magnetic sources, e.g. motors, or objects, e.g. body parts of the patient 5 to be imaged.

In another embodiment, the processing unit 30 is configured to determine less than nine, in particular six, distances between the three first distance sensors 12a, 12b, 12c and the three third distance sensors 13a, 13b, 13c.

In another embodiment, the processing unit 30 is further configured to determine the position and/or distance of the radiation generation unit 2 and the detection unit 6 relative to each other based on less than nine, in particular six, distances between the three first distance sensors 12a, 12b, 12c and the three second distance sensors 13a, 13b, 13c and the, in particular fixed, distances between each of the first 12a, the second 12b and the third 12c of the first distance sensors 12a, 12b, 12c and/or the, in particular fixed, distances between each of the first 12a, second 12b and the third 13c of the second distance sensors 13a, 13b, 13c. In particular, the processing unit 30 may be configured in this way even though more than six, in particular nine, distances between the three first distance sensors 12a, 12b, 12c and the three second distance sensors 13a, 13b, 13c are determined. This embodiment is particularly preferable if the determination of some of the more than six, in particular nine, determined distances is influenced by external fields, e.g. generated by motors nearby, or objects located between the several distance sensors hampering the one or more signal emitted and received by the several distance sensors. The same is valid for embodiments with more than three first distance sensors 12a, 12b, 12c and more than three second distance sensors 13a, 13b, 13c, where more than nine distances between the several sensors can be determined.

Regarding further preferred features of the processing unit 30 shown in Figure 6, the third sensor unit 44, the output unit 30, the control element 32, the drive unit 33 and the movement unit 34, the above elucidations with reference to Figure 3 apply accordingly.

**Claims**

1. A mobile radiation image capturing system (20) comprising:

   - a radiation generation unit (2) configured to generate X-ray radiation (4);
   - a mobile carriage (7, 21) on which the radiation generation unit (2) is mounted;
   - a drive unit (33) configured to move the carriage (7, 21) over the floor (15) by linear motion in two dimensions and/or by rotation;
   - at least one detection unit (6) configured to capture a radiation image of an object (5) based on X-ray radiation (4) generated by the radiation generation unit (2) and transmitted and/or reflected by the object (5);
   - at least one sensor unit (10, 11) configured to determine an orientation and/or a position of the radiation generation unit (2) and the detection unit (6) relative to each other,
   - at least one output unit (31) configured to output information to a user,
   - a processing unit (30) configured to determine, based on the orientation and/or position of the radiation generation unit (2) and the detection unit (6) relative to each other, whether the radiation generation unit (2) and the detection unit (6) have a predetermined orientation and/or position relative to each other and, in the negative, to determine a positioning information on how the carriage (7, 21) and/or the radiation generation unit (2) has to be moved in order to bring the radiation generation unit (2) and the detection unit (6) into a predetermined orientation and/or position relative to each other, and to control the output unit (31) to output the determined positioning information and/or to control the drive unit (33) to move the carriage (7, 21) based on the determined positioning information.

2. The mobile radiation image capturing system (20) according to claim 1 comprising at least one control element (32) configured to receive movement instructions from the user, wherein the processing unit (30) is configured to control the drive unit (33) to move the carriage (7, 21) based on the movement instructions received from the user.

3. The mobile radiation image capturing system (20) according claim 2, wherein the at least one control element (32) is located at the radiation generation unit (2), in particular at a housing enclosing the radiation generation unit (2).

4. The mobile radiation image capturing system (20) according claim 2 or 3, wherein the at least one output unit (31) is located close to the at least one control element (32) and/or at the radiation generation unit (2), in particular at the housing enclosing the radiation generation unit (2).

5. The mobile radiation image capturing system (20) according to any of the preceding claims, wherein the at least one output unit (31) is configured to visually and/or acoustically output information.

6. The mobile radiation image capturing system (20) according to any of the preceding claims, further comprising a first sensor unit (10) provided at the radiation generation unit (2) and a second sensor unit (11) provided at the detection unit (6), wherein each of the first and second sensor unit (10, 11) comprises at least one of the following:

- at least one accelerometer sensor (41) configured to capture first information regarding an acceleration of the accelerometer sensor (41) with respect to three spatial directions,
- at least one gyroscope (42) sensor configured to capture second information regarding an orientation of the gyroscope sensor (42) with respect to the three spatial directions, and
- at least one magnetic field sensor (43) configured to capture third information regarding a magnetic field surrounding the magnetic field sensor (43) along the three spatial directions,

wherein the processing unit (30) is configured to determine the orientation and/or position of the radiation generation unit (2) and the detection unit (6) relative to each other by considering

- at least one of the first, second and third information captured by the accelerometer (41), gyroscope (42) or magnetic field sensor (43), respectively, of the first sensor unit (10) provided at the radiation generation unit (2), and
- at least one of the first, second and third information captured by the accelerometer (41), gyroscope (42) and magnetic field sensor (43), respectively, of the second sensor unit (11) provided at the detection unit (6).

7. The mobile radiation image capturing system (20) according to claim 6 comprising

- at least one transmitter (12) configured to emit one or more signals and at least one receiver (13) configured to receive the signals emitted by the transmitter (12), the transmitter (12) or receiver (13) being provided at the radiation generation unit (2), and the receiver (13) or transmitter (12), respectively, being provided at the detection unit (6); and
- a processing unit (30) configured to determine a position of the radiation generation unit (2) and the detection unit (6) relative to each other by considering the signals received by the receiver (13) and at least one of the first, second and third information captured by the accelerometer (41), gyroscope (42) and magnetic field sensor (43), respectively, of the first sensor unit (10) provided at the radiation generation unit (2), and at least one of the first, second and third information captured by the accelerometer (41), gyroscope (42) and magnetic field sensor (43), respectively, of the second sensor (11) unit provided at the detection unit (6).

8. The mobile radiation image capturing system (20) according to any of the claims 1 to 5, comprising

- at least three transmitters (12) configured to emit one or more signals and at least three receivers (13) configured to receive the signals emitted by the transmitter (12), the at least three transmitters (12) or at least three receivers (13) being provided at the radiation generation unit (2), and the at least three receivers (13) or at least three transmitters (12), respectively, being provided at the detection unit (6); and
- a processing unit (30) configured

to determine distances between the at least three transmitters (12) and the at least three receivers (13),
to determine an orientation of the radiation generation unit (2) and the detection unit (6) relative to each other by considering the determined distances between the at least three transmitters (12) and at least three receivers (13), and
to determine a position, in particular a distance, of the radiation generation unit (2) and the detection unit (6) relative to each other by considering the determined distances between the at least three transmitters (12) and the at least three receivers (13) and the determined orientation of the radiation generation unit (2) and the detection unit (6) relative to each other.

9. The radiation image capturing system (20) according to claim 7 or 8, wherein the one or more signals emitted by the at least one transmitter (12) and received by the at least one receiver (13) are ultrasound signals or magnetic signals, in particular a magnetic flux, or electromagnetic signals, in particular light.

10. The mobile radiation image capturing system (20) according to any of the preceding claims, further comprising a third sensor unit (44) configured to determine an inclination of the carriage (7, 21) with respect to the vertical and/or horizontal direction, wherein the processing unit (30) is configured to control the drive unit (33) to move the carriage (7, 21) based on the determined inclination of the carriage (7, 21).

11. A method for operating a mobile radiation image capturing system (20), the system comprising a radiation generation unit (2) configured to generate X-ray radiation (4), a mobile carriage (7, 21) on which the radiation generation unit (2) is mounted, a drive unit (33) configured to move the carriage (7, 21) over the floor, and at least one detection unit (6) configured to capture a radiation image of an object (5) based on X-ray radiation (4) generated by the radiation generation unit (2) and transmitted and/or reflected by the object (5), the method comprising the following steps:

   - determining an orientation and/or a position of the radiation generation unit (2) and the detection unit (6) relative to each other,
   - determining, based on the orientation and/or position of the radiation generation unit (2) and the detection unit (6) relative to each other, whether the radiation generation unit (2) and the detection unit (6) have a predetermined orientation and/or position relative to each other and, in the negative, determining a positioning information on how the carriage (7, 21) and/or the radiation generation unit (2) has to be moved in order to bring the radiation generation unit (2) and the detection unit (6) into a predetermined orientation and/or position relative to each other, and outputting the positioning information via an output unit (31) to a user and/or controlling the drive unit (33) to move, based on the positioning information, the carriage (7, 21) over the floor (15) by linear motion in two dimensions and/or by rotation.

Fig. 1

Fig. 2

Fig. 3

Fig. 4

EP 3 235 432 A1

Fig. 5

Fig. 6

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION  (IPC) |
|---|---|---|---|
| X | US 2014/247918 A1 (KANG DONG GOO [KR] ET AL) 4 September 2014 (2014-09-04) | 1-7,9,11 | INV. A61B6/00 |
| Y | * paragraph [0072] - paragraph [0138]; figures * | 8,10 | |
| | ----- | | |
| X | DE 10 2010 008552 A1 (SIEMENS AG [DE]) 25 August 2011 (2011-08-25) | 1,4-6,11 | |
| Y | * paragraph [0007] - paragraph [0037]; figures * | 8 | |
| | ----- | | |
| Y | JP 2009 022677 A (SHIMADZU CORP) 5 February 2009 (2009-02-05) * paragraph [0001] - paragraph [0017]; figures * | 10 | |
| | ----- | | |
| Y | JP 2010 207393 A (SHIMADZU CORP) 24 September 2010 (2010-09-24) * paragraph [0035] - paragraph [0036]; figures * | 10 | |
| | ----- | | **TECHNICAL FIELDS SEARCHED      (IPC)** A61B |

The present search report has been drawn up for all claims

| Place of search | Date of completion of the search | Examiner |
|---|---|---|
| Munich | 22 September 2016 | Strubel, Christine |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 16 16 5956

This annex lists the patent family members relating to the patent documents cited in the above-mentioned European search report.
The members are as contained in the European Patent Office EDP file on
The European Patent Office is in no way liable for these particulars which are merely given for the purpose of information.

22-09-2016

| Patent document cited in search report | Publication date | Patent family member(s) | Publication date |
|---|---|---|---|
| US 2014247918 A1 | 04-09-2014 | CN 104027122 A<br>EP 2774541 A1<br>JP 2014168690 A<br>KR 20140108989 A<br>US 2014247918 A1 | 10-09-2014<br>10-09-2014<br>18-09-2014<br>15-09-2014<br>04-09-2014 |
| DE 102010008552 A1 | 25-08-2011 | NONE | |
| JP 2009022677 A | 05-02-2009 | NONE | |
| JP 2010207393 A | 24-09-2010 | JP 5077270 B2<br>JP 2010207393 A | 21-11-2012<br>24-09-2010 |

EPO FORM P0459

For more details about this annex : see Official Journal of the European Patent Office, No. 12/82